Europäisches Patentamt

European Patent Office

Office européen des brevets

(19)

(11) Publication number: **0 318 301**
**A2**

## (12) EUROPEAN PATENT APPLICATION

(21) Application number: **88311157.7**

(22) Date of filing: **25.11.88**

(51) Int. Cl.⁴: **C 12 Q 1/68**
**C 12 N 15/00**

(30) Priority: **25.11.87 US 125577   16.11.88 US 271653**

(43) Date of publication of application:
**31.05.89 Bulletin 89/22**

(84) Designated Contracting States: **DE FR GB**

(71) Applicant: **CITY OF HOPE**
**1500 East Duarte Road**
**Duarte California 91010-0269 (US)**

(72) Inventor: **Comings, David Edward**
**59 Crestview Court**
**Duarte California 91010 (US)**

(74) Representative: **Hallybone, Huw George**
**CARPMAELS AND RANSFORD 43 Bloomsbury Square**
**London WC1A 2RA (GB)**

(54) **Tourette syndrome, autism and associated behaviours.**

(57) Diagnosis of genetic Tourette syndrome and related psychiatric and behavioral disorders by genetic tests to identify deletions or defective alleles in the human tryptophan oxygenase (TO) gene and to cDNA probes for use in such tests.

EP 0 318 301 A2

Bundesdruckerei Berlin

**Description**

## TOURETTE SYNDROME, AUTISM AND ASSOCIATED BEHAVIORS

### FIELD OF THE INVENTION

This invention relates to the diagnosis of genetic Tourette syndrome and related psychiatric and behavioral disorders by genetic tests to identify deletions, duplications or defective alleles in the human tryptophan oxygenase (TO) gene and to probes for use in such tests. More particularly, the invention relates to certain cDNA tryptophan oxygenase clones derived from mRNA and to the use of such clones as probes to identify genomic clones from a human genomic library and to identify the chromosomal location of the human TO gene.

Diagnostic tests are provided for, among other things, tourette syndrome (TS) chronic motor and vocal tics, attention deficit disorder (ADD) with or without hyperactivity, obsessive-compulsive behaviour, unipolar or bipolar affective disorder, phobias, panic attacks, generalized anxiety disorder, autism, pervasive developmental disorder, dyslexia, learning disabilities, dysgraphia, borderline personality disorder, exhibitionism, stuttering, delayed speech, schizophreniform disorder, schizoid disorder, drug or alcohol addiction, bulimia, compulsive eating with obesity, physical or sexual abuse of spouse or children, and severe mental retardation (MR). The fact that TS is a wide-based, generalized behavioral disorder associated with the above list of abnormalities is documented in Comings, D.E. and Comings, B.G. "A controlled study of Tourette syndrome. I. Attention deficit disorder, learning disorders and school problems", Amer. J. Human Genetics 41:701-741 (1987); Comings, D.E. and Comings, B,G., "A controlled study of Tourette syndrome. II. Conduct disorder", Amer. J. Human Genetics 41:742-760 (1987); Comings, D.E. and Comings, B.G., "A controlled study of Tourette syndrome. III. Phobias and panic attacks", Amer. J. Human Genetics 41:761-781 (1987); Comings, D.E. and Comings, B.G., "A controlled study of Tourette syndrome. IV. Obsessive compulsive and schizoid behavior", Amer. J. Human Genetics 41:782-803 (1987); Comings, D.E. and Comings, B.G., "A controlled study of Tourette syndrome. V. Depression and mania", Amer. J. Human Genetics 41:804-821 (1987); Comings, D.E. and Comings, B.G., "A controlled study of Tourette syndrome. VI. Early development, sleep problems, allergies and handedness", Amer. J. Human Genetics 41:822-838 (1987). Hereinafter these abnormalities are collectively referenced as "TS related disorders".

### DETAILED DESCRIPTION OF THE INVENTION

Tourette syndrome (TS) is a common, hereditary, neuro-behavioral disorder characterized by motor and vocal tics. Autism is a trait of three sets of symptoms: (1) a professed failure to develop social relationships; (2) defective speech and language; and (3) ritualistic or compulsive behaviour.

The symptoms of TS and its related disorders fit a model in which the frontal lobe and limbic system in the brain are disinhibited by a relative deficiency in brain serotonin and tryptophan.

Like TS, autism and MR are neurological disorders. The areas of the brain involved in autism and MR, the frontal lobe and limbic system, are often the same as those involved in TS. Unlike TS where both brain serotonin and tryptophan levels tend to be low, in autism and MR each is often significantly elevated.

It appears that serotonin and tryptophan levels are interdependent, serotonin level abnormalities being a consequence of tryptophan level abnormalities. Since tryptophan is an essential amino acid, all the tryptophan in the body comes from diet or gut bacteria. Diagram 1 shows the two major pathways it can take, i.e., 90% in the formation of kynurenine and 10% in the formation of serotonin.

tryptophan

| 10% | | 90% |
Tryptophan hydroxylase | | | Tryptophan oxygenase

5-hydroxytryptophan      N-formyl kynurenine

L-aromatic acid decarboxylase | | | Kynurenine formylase

serotonin          kynurenine

## DIAGRAM 1

The rate limiting step is the 90% degradation of tryptophan to kynurenine is TO activity. Thus fairly subtle changes in TO level would be expected to significantly affect serotonin production. TO is thus implicated in both TS and autism. The level of the enzyme is proposed to be increased in TS but decreased in autism, a reciprocal disorder.

Tryptophan-2,3 dioxygenase (tryptophan oxygenase, tryptophan pyrrolase) is considered primarily to be a liver enzyme. However, the same or a very similar enzyme, indoleamine 2,3 dioxygenase is also present in the brain, intestines and other organs. Gal, E.M. and Sherman, A.D. "L-kynurenine: its synthesis and possible regulatory function in brain", Neurochem. Res. 5: 223-239 (1980). Table I depicts several differences between the functions of these enzymes. Hayaishi, O., "Properties and function of indoleamine 2,3-dioxygenase", J. Biochem 79:13p-21p (1976); Gal, E.M., "Cerebral tryptophan-2,3-dioxygenase (pyrrolase) and its induction in rat brain" J. Neurochem 22:861-863 (1974); and Gal, E.M. and Sherman, A.D. "L-kynurenine: its synthesis and possible regulatory function in brain", Neurochem. Res. 5: 223-239 (1980).

### TABLE 1

| | Tryptophan 2,3-dioxyge-nase | Indoleamine 2,3-dioxyge-nase |
|---|---|---|
| Similarities | | |
| Substrate | L-tryptophan | L-tryptophan |
| Product | N-formyl kynurenine | N-formyl kynurenine |
| Type of protein | heme | heme |
| Inducible by tryptophan | yes | yes |
| Differences | | |
| Location | liver | brain and many other organs |
| Type of oxygen used | $O_2$ | $O\text{-}O^-$ |
| Other substrates | none | D-tryptophan 5-hydroxytryptophan serotonin melantoin |
| Inducible by cortisol | yes | no |

Whether these differences are due to the presence of the enzyme in different tissues or to the fact that the enzymes are produced by different genes is not known. For the purposes of this invention, it is unnecessary to determine whether one or two genes is implicated. Unless separately identified, the liver and brain-intestine enzymes are referenced collectively herein as tryptophan oxygenase or TO.

If TO were present only in the liver, then the level of tryptophan in the brain would be almost completely dependent on the blood level of tryptophan and of the compounds that compete for its transport across the blood brain barrier. However, as Diagram 2 shows, tryptophan oxygenase activity is also present in the brain where it is effective to siphon off some of the tryptophan after brain entry and before it is converted to serotonin.

```
Blood                       Brain

tryptophan  ──────→   tryptophan ──→   ──→   serotonin
                              \      brain tryptophan
                               \          oxygenase
                                \↘
                                  ──────→    kynurenine
```

DIAGRAM 2

In rats, under normal conditions 70% of the brain tryptophan is converted to serotonin and 30% to brain kynurenine. Gal, E.M. and Sherman, A.D., "L-kynurenine: its synthesis and possible regulatory function in brain", Neurochem. Res. 5:233-239 (1980). Even a moderate increase in brain tryptophan oxygenase could markedly change this ratio and lower the level of brain serotonin. Significant changes in the breakdown of tryptophan in the brain may thus occur concurrently with only moderate changes in the blood tryptophan and serotonin.

Diagram 3 depicts a focal postulate of this invention, i.e., that the genetic defect in TS related disorders is either a duplication or a mutation of the TO gene resulting in high levels of hyperinducability, whereas in autism and some forms of MR the TO gene is either defective or deleted.

4

Defective Brain-Intestine TO Gene

Abnormally Active Brain-Intestine TO

↓ decreased brain tryptophan

↑ increased breakdown of serotonin to
5-hydroxykynurenine

↓ decreased serotonin in brain and platelets

TS symptoms

disinhibition of
striatial dopamine
neurons

motor and vocal
hyperactivity

response to
dopamine and
norepinephrine
in the frontal
lobe

frontal lobe
syndrome
learning dis-
orders
ADDH

behavioral
disinhibition

aggression, conduct disorder,
drug and alcohol abuse, heat
intolerance, EEG abnormalities,
hyperactivity, mood disorder,
obsessive compulsive, resistance
to discipline, schizoid symptoms,
sensitive to sensations, sexual
disinhibition, short temper,
sleep disorders

Abnormally Inactive Brain-Intestine TO

↑ increased serum tryptophan

↑ increased serotonin in brain and platelets

inhibition
of some
neurons

compensatory
disinhibition
of some
neurons

stimulation
of some
endorphins

autism or severe mental retardation

DIAGRAM 3

EP 0 318 301 A2

As shown by Diagram4, a decrease in brain serotonin contributing to TS is also enhanced by an abnormally active liver form of TO.

```
           Abnormally Active Liver TO

                        │
                        ↓

   ↓ decreased serum tryptophan
      (due to TO breakdown to kynurenine)

                        │
                        ↓

   ↑ increased serum kynurenine
      (competes with tryptophan for transport into the brain)

                        │
                        ↓

   ↓ decreased brain tryptophan

                        │
                        ↓

   ↓ decreased brain serotonin

                        │
                        ↓

       TS symptom spectrum
```

## DIAGRAM 4

Consistent with its focal postulate, this invention provides genetic tests for TS, autism and related disorders which entail the identification of deletions, duplications and other defective alleles of the TO gene.

These genetic tests, per se, may be of conventional nature and involve, for example:

(a) Obtaining DNA from subjects to be tested (blood, skin, hair follicles or any other source).

(b) Identifying mutations in this DNA by hybridization of specific DNA or RNA probes to various parts of the tryptophan oxygenase gene, or DNA surrounding the tryptophan oxygenase gene. The mutations may be partial or complete gene duplications, double or multiple gene duplications, partial or complete gene deletions, single nucleotide substitutions, or frame shift mutations. The probes may, for example, be oligonucleotide probes including allele specific synthetic oligonucleotides, cloned cDNA, genomic DNA fragments, or RNA. The test may include hybridization of DNA electrophoresed in gels, or spotted on a support media, or amplified by DNA polymerases.

The invention includes a cloned cDNA and genomic DNA probe for the human TO gene useful to conduct such genetic tests.

## Cloning of The Human TO Gene

Schmidt, et al. Schmidt, W., Scherer, G., Danesch, U., Zentgraf, H., Matthias, P., Strange, C.M., Towekamp, W. and Schutz, G., "Isolation and characterization of the rat tryptophan oxygenase gene. EMBO Journal 1:1268-1287 (1982). report the isolation of the rat liver tryptophan oxygenase gene. Two cDNA probes, pcTO1 and pcTO2 covering parts of the entire rat TO gene are described. pcTO1 and pcTO2 probes obtained from Schmidt did not hybridize well to human DNA and hence were not useful for the type of genetic tests contemplated by this invention.

To provide an appropriate cDNA probe, the rat pcTO1 clone was labelled and hybridized to a Clonetech Clonetech Laboratories, Inc., Human Liver cDNA, Library Catalogue No. HL 101B, Lot No. 2102. human liver cDNA library in λGT11 containing several hundred thousand liver cDNA clones. Duplicate lifts on nitrocellulose filter papers were made with each petri dish that contained about 30,000 plaques. Non-specific artifacts were ruled out if a positive signal (dark spot on the autoradiogram) was obtained in the same position on each duplicate lift as determined by superimposing the two autoradiograms in a slightly shifted position and examination for double spots.

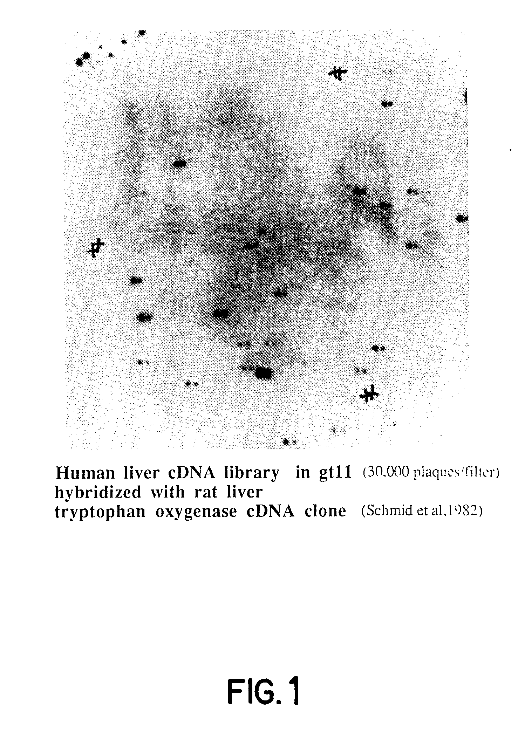

Figure 1, from just one of nine different dishes, depicts autoradiograms of duplicate filter lifts superimposed and turned slightly. Double spots (duplicate hybridizations) each represent a cDNA clone of human tryptophan oxygenase. The +'s are orientation markers.

As this data shows, the human tryptophan oxygenase gene has been isolated, and cDNA clones useful as or to provide, in a manner known to persons skilled in the art, probes for the conduct of genetic tests pursuant to the invention have been identified. While cDNA clone was derived from a liver library, in view of the close similarity, if not identity, of the liver and brain-intestine TO genes, such probes may be used with respect to both.

## EXAMPLE I

### Preparation of 2000 bp Human cDNA to Tryptophan Oxygenase

A Clonetech cDNA library to human liver messenger RNA, in λGT11, was plated on LB at a density of 30,000 pfu, using LE 192 hose E. coli. Plaques were lifted in duplicate on BA85 circles, washed in 0.5 N NaOH, 1.5 M NaCl, then 0.5 M Tris, pH 8, 1.5 M NaCl, then baked 2 hours at 80°C. The filters were then prehybridized 2 hours in 6XX SSC, 1X Denhardts, 100 ug/ml salmon sperm DNA at 65°C, 0.2 ml/sq.cm.

The pcTO1 insert DNA was labelled with 32p by nick translation and hybridized overnight in 6X SSC, 1X Denhardts, 0.1% SDS, 65°C, then washed in 2X SSC, 1X Denhardts at room temperature for 30 min, then twice in 0.3X SSC, 0.5% SDS, 65°C for 1 hour. The plaques that were positive on both duplicate plaque lifts were identified and replated at low density and final single plaques grown up of each positive clone. These represented cDNA clones of human liver messenger RNA that gave a hybridization signal cDNA clones to rat liver tryptophan oxidase.

## EXAMPLE II

### Isolation of Human Genomic TO Clones

A human genomic library Fritsch, E.F., Lawn, R.M. and Maniatis, T.: Molecular cloning and characterization of the human β-like globin gene cluster, Cell 19:959-972 (1980). in phage λ Charon 4a was hybridized with the pHTO3 clone described in Example I. Hybridization identified 12 genomic clones. Each genomic clone was isolated and cut by digestion with EcoRI restriction enzyme.

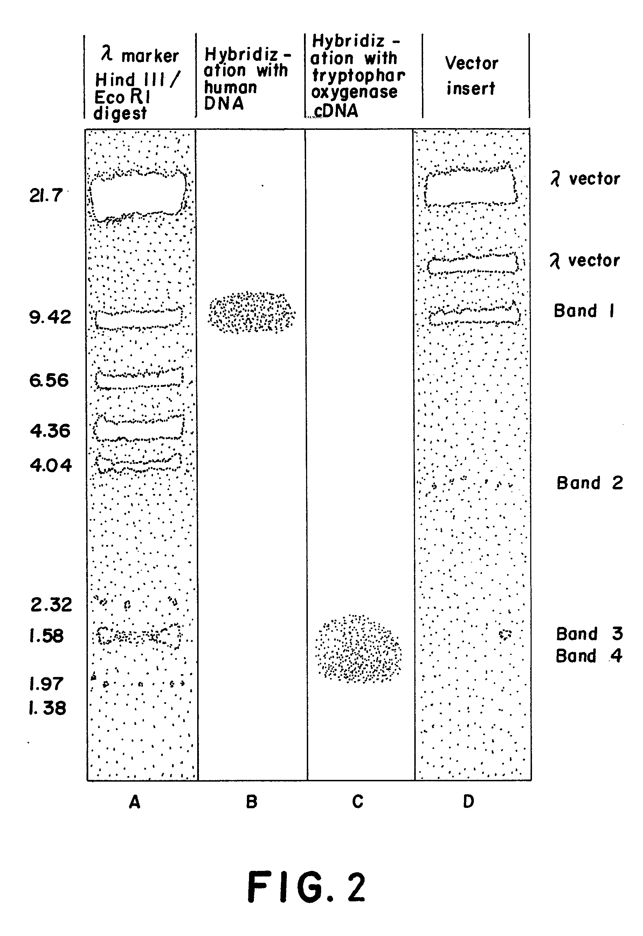

Lane D of Figure 2 shows the results of gel electrophoresis of the cut λ vector. Four bands totalling an insert of approximately 16,000 base pairs are apparent.

A λ phage containing human genomic sequences that hybridized to the pHTO3 probe was digested with HindIII, electrophoresed in 1% agarose, and transferred by Southern blotting to Nytran filters. The pHTO3 probe was labelled with 32p by nick translation. The labelled probe was hybridized to the Southern blot and autoradiographed with XAR-S with Lightning+ screen.

Hybridization showed that band 4 DNA of almost 2,000 base pairs contained most of the sequences homologous to a pHTO3 probe (lane C) and shows slight hybridization of the DNA of band 3 with the pHTO3 probe.

Lane B of Figure 2 shows that the DNA of band 1, when hybridized against 32p labelled total human DNA, appears to contain repetitious DNA sequences present in the human genome.

Lane A of Figure 2 shows the result of gel electrophoresis of mixed marker of HindIII and EcoRI λ digests.

### Chromosomal Location of The Human TO Gene

Example III establishes that the human TO gene is located on the short arm of chromosome 4 between band q25 and the tip or telomere.

## EXAMPLE III

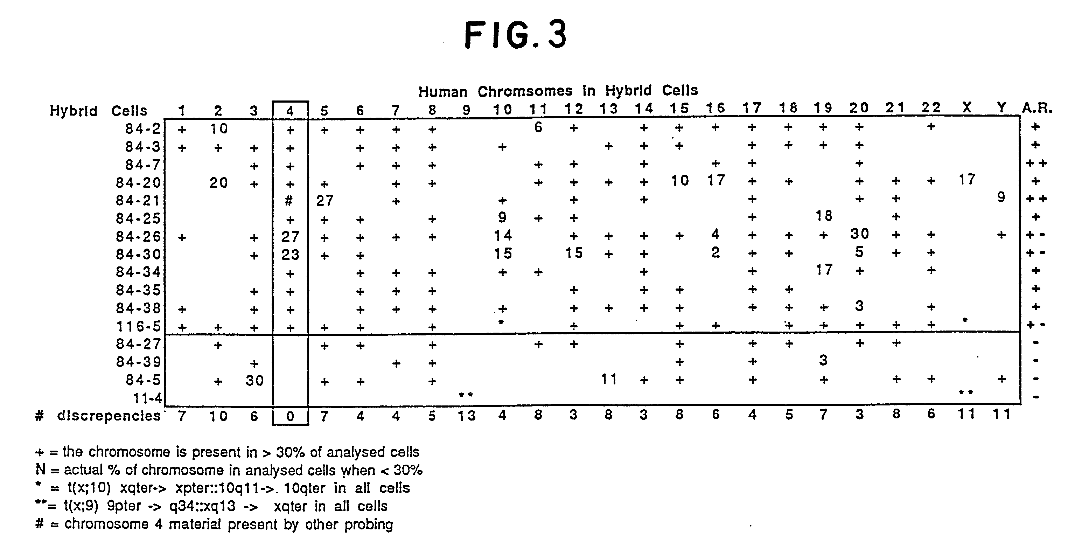

DNA from a set of 16 Chinese hamster cell hybrids containing varying amounts of human chromosomes was obtained from Dr. Mohandas, the Department of Medical Genetics of the University of California at Los Angeles (UCLA). These 16 samples of DNA were cut with a restriction endonuclease, specifically Puv II, electrophoresed and Southern blotted on a nylon filter. The DNA restriction fragments were then hybridized with $^{32}$p labelled pHTO3. Figure 3 shows the results. The column labelled A.R. shows the results of the hybridization to the human DNA where $++$ to $+-$ indicates positive hybridization and $-$ represents no hybridization. No discrepancies were observed for chromosome #4, thus indicating that the pHTO3 sequences were on chromosome #4.

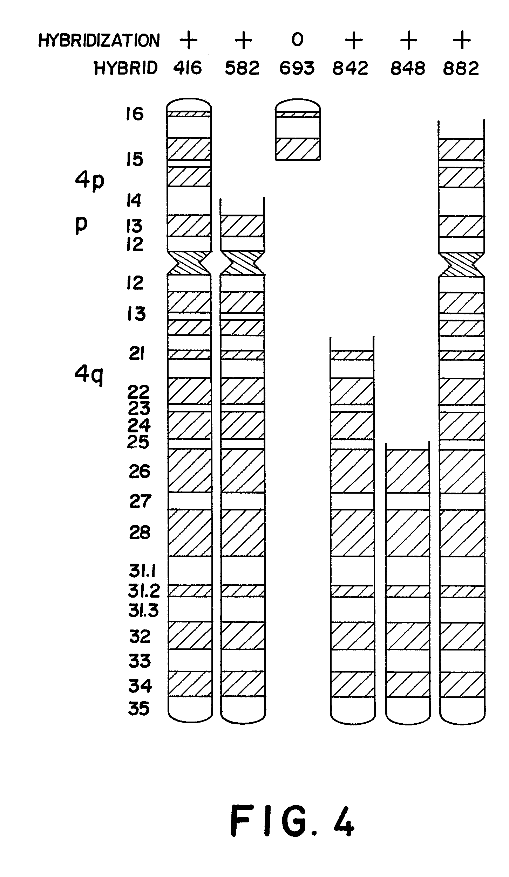

A different set of DNA from hybrid Chinese hamster cells containing varying amounts of human chromosome #4 was obtained from Dr. John Wasmuth of the University of California at Irvine. A similar series of cutting with the restriction endonuclease Puv II, electrophoreses, Southern blotting and hybridization was performed. The results of the hybridization are depicted by Figure 4. In the figure, the numbers 416, 582, 693, 842, 848 and 882 indicate hybrid DNA fragments. Inasmuch as 693, which contained only the tip of the short arm of chromosome #4 was negative, the gene for pHTO3 was not on this part of the chromosome. Since all the other hybridizations were positive but varying amounts of chromosome #4 were missing from several of these cell hybrids, Figure 4 shows the gene to pHTO3 to be on 4q between q25 and q telomere (hybrid 848).

Implication of two, albeit equivalent or identical, TO genes affords an explanation for the behavioral and psychiatric disorders consequent from abnormal levels of brain serotonin.

Since the serotonin levels in some severe mental retardation are even higher than those in autism, it is likely that the genetic defect causing the elevated serotonin is also more severe and may represent a deletion of the TO gene. By contrast, in TS, the TO genes are duplicated or otherwise mutated so the amount of enzyme is increased.

In genetic terms the most detrimental thing that can be done to a gene is to delete it. A single base change mutation within the gene may have no effect, a mild effect or a significant effect on the function of the enzyme that gene makes. For the purposes of the following Table 2 the liver and brain-intestine genes are deemed equivalent. A deletion of a TO gene will be termed TO 0, a base pair substitution mutation will be termed TO -, a normal gene will be termed TO + and a duplicated gene, or a gene with a mutation causing increased levels or increased efficiency of the tryptophan oxygenase enzyme, as TO $++$.

TABLE 2

| Type of Defect | Genotype | Effect on Blood Serotonin | Result |
|---|---|---|---|
| None | TO +/TO + | Normal Levels | Normal |
| Deleted/ Deleted | TO O/TO O | Extremely High | Severe M.R. |
| Deleted/ Mutant | TO O/TO - | Very High | Autism with M.R. |
| | TO O/TO | | |
| Mutant/ Mutant | TO -/TO - | High | Autism |
| Deleted/ Normal | TO O/TO + | Moderately High | Normal or Mild Autism |
| Dup/Normal Normal | TO $++$/TO + | Low Serotonin | Tourette Syndrome |
| Dup/Dup | TO $++$/TO $++$ | Low Serotonin | Homozygous Grade 3 TS |

It is thus suggested that mutations of the human TO gene which result in decreased activity of the TO gene are likely to be involved in some cases of the autism or autism with mental retardation. These mutations would be various heterozygous or homozygous combinations of deletions or base pair mutations affecting activity or function of the human TO gene or genes.

It is suggested that mutations of the human TO gene which result in increased activity, or increased inducability or increased efficiency of the TO gene are likely to be involved in Tourette syndrome and many of the wide range of behaviors associated with the TS spectrum of behaviors. These mutations would be various heterozygous or homozygous combinations of gene duplications, or mutations of the regulatory or structural sequences of the human TO gene or genes.

Because the defects are enzyme deficiencies, the autism and MR disorders are autosomal recessive. By contrast, for TS because the enzyme level is apparently increased, the trait is autosomal dominant or co-dominant. This is consistent with the observation that the administration of large amounts of vitamin B6 or pyridoxine improves the symptoms in autistic patients. Rimland, B., Callaway, E. and Dreyfus, P., "The effect of high doses of vitamin B$_6$ on autistic children: A double-blind crossover study", Am. J. Psychiatry 135:472-475 (1978). Vitamin B$_6$ is a cofactor for many of the enzymes involved in the kynurenine pathway.

**Claims**

1. A procedure for diagnosis of Tourette syndrome, autism, or severe mental retardation which comprises identification of deletions, duplications or defective alleles in a human tryptophan oxygenase gene by a genetic test conducted on a sample from a subject to be tested.

2. The procedure as defined in claim 1 in which the human tryptophan oxygenase gene expresses tryptophan 2,3-dioxygenase.

3. The procedure as defined in claim 1 in which the human tryptophan oxygenase gene expresses indoleamine 2,3-dioxygenase.

4. A procedure as defined by claim 1 in which the genetic test comprises identification of a deletion of or a defective allele in a human tryptophan oxygenase gene by hybridizing a specific DNA or RNA probe to at least a part of said gene.

5. A procedure as defined in claim 4 in which the probe is a cDNA probe, a genomic probe, an allele specific synthetic oligonucleotide or pHTO3.

6. A procedure as defined in claim 1 for the diagnosis of Tourette syndrome by a genetic test to identify a defective allele in a human tryptophan oxygenase gene resulting in high levels or hyperinducability of TO or increased efficiency pTO and low brain serotonin level.

7. A procedure as defined in claim 1 for the diagnosis of autism by a genetic test to identify a deleted or defective human tryptophan oxygenase gene resulting in a high brain serotonin level.

8. A procedure as defined by claim 7 in which the genetic test comprises the use of an allele specific synthetic oligonucleotide probe.

9. A cDNA clone of a human tryptophan oxygenase gene.

10. An isolated human tryptophan oxygenase gene.

11. The human tryptophan oxygenase clone pHTO3.

12. A human, genomic TO clone which, when cut by EcoRI yields a gene having a gel electrophoresis pattern having a band of approximately 2,000 base pairs substantially corresponding to so much of the pattern of Lane D of Figure 2 as includes band 1 or band 4 or band 1 and band 4.

Human liver cDNA library  in gt11 (30,000 plaques/filter)
hybridized with rat liver
tryptophan oxygenase cDNA clone  (Schmid et al.1982)

FIG. 1

FIG. 2

# FIG. 3

Human Chromsomes In Hybrid Cells

| Hybrid Cells | 1 | 2 | 3 | 4 | 5 | 6 | 7 | 8 | 9 | 10 | 11 | 12 | 13 | 14 | 15 | 16 | 17 | 18 | 19 | 20 | 21 | 22 | X | Y | A.R. |
|---|---|---|---|---|---|---|---|---|---|---|---|---|---|---|---|---|---|---|---|---|---|---|---|---|---|
| 84-2 | + | 10 | | + | + | + | + | + | | | 6 | + | | + | + | + | + | + | + | + | | + | | | + |
| 84-3 | + | + | + | + | | + | + | + | | + | | | + | + | + | | + | + | + | + | | | | | + |
| 84-7 | | | + | + | | + | + | + | | | + | + | | + | | + | + | | | + | | | | | ++ |
| 84-20 | | 20 | + | + | + | | + | + | | | + | + | + | + | 10 | 17 | + | + | | + | + | + | 17 | | + |
| 84-21 | | | | # | 27 | | + | | | + | | + | | + | | | + | | | + | + | | | 9 | ++ |
| 84-25 | | | | + | + | + | | + | | 9 | + | + | | | | | + | | 18 | | + | | | | + |
| 84-26 | + | | + | 27 | + | + | + | + | | 14 | | + | + | + | + | 4 | + | + | + | 30 | + | + | | + | +- |
| 84-30 | | | + | 23 | + | + | | | | 15 | | 15 | + | + | | 2 | + | + | | 5 | + | + | | | +- |
| 84-34 | | | | + | | + | + | + | | + | + | | | + | | | + | | 17 | + | | + | | | + |
| 84-35 | | | + | + | | + | + | + | | | | + | | + | + | | + | + | | | | | | | + |
| 84-38 | + | | + | + | | + | + | + | | + | | + | + | + | + | | + | + | + | 3 | | + | | | + |
| 116-5 | + | + | + | + | + | + | | + | * | | | + | | + | | + | | + | + | + | + | + | * | | +- |
| 84-27 | | + | | | + | + | | + | | | + | + | | | + | | + | + | | + | + | | | | - |
| 84-39 | | | + | | | | + | + | | | | | | | + | | + | | 3 | | | | | | - |
| 84-5 | | + | 30 | | + | + | | + | | | | | 11 | + | + | | + | | + | | + | + | | + | - |
| 11-4 | | | | | | | | ** | | | | | | | | | | | | | | | ** | | - |
| # discrepencies | 7 | 10 | 6 | 0 | 7 | 4 | 4 | 5 | 13 | 4 | 8 | 3 | 8 | 3 | 8 | 6 | 4 | 5 | 7 | 3 | 8 | 6 | 11 | 11 | |

+ = the chromosome is present in > 30% of analysed cells
N = actual % of chromosome in analysed cells when < 30%
* = t(x;10) xqter-> xpter::10q11->. 10qter in all cells
**= t(x;9) 9pter -> q34::xq13 -> xqter in all cells
# = chromosome 4 material present by other probing

EP 0 318 301 A2

FIG. 4